# EUROPEAN PATENT APPLICATION

(11) **EP 3 375 888 A1**
(43) Date of publication of application: **19.09.2018**
(21) Application number: 17305294.5
(22) Date of filing: 17.03.2017
(51) Int. Cl.: C12Q 1/68, G01N 33/574

(54) **A METHOD FOR PREDICTING THE RESPONSIVENESS OF A PATIENT TO A TREATMENT WITH MTOR INHBITORS**

(71) Applicant: INSERM (Institut National de la Santé et de la Recherche Médicale), 75013 Paris (FR); Université De Nice Sophia Antipolis, 06103 Nice (FR)
(72) Inventor: RICCI, Jean-Ehrland, 06204 Nice (FR); THIEBLEMONT, Catherine, 75014 Paris (FR); CHICHE, Johanna, 06204 Nice (FR)
(74) Representative: Hirsch, Denise Marie

(57) **Abstract**

The present invention relates to a method for predicting the responsiveness of a patient to a treatment with mtor inhbitors. Using primary Eµ-Myc lymphoma cells, inventors observed that Eµ- Myc-GAPDHhigh clones presented less mTOR activity than Eµ-Myc-GAPDHlow clones, as determined by the increase in p70-S6K phosphorylation in the latter. Importantly, inhibition of mTOR with rapamycin in two independent Eµ- Myc-GAPDHlow clones induces a metabolic shift from OxPhos to glycolysis. These results suggest that GAPDH-dependent modulation of the mTOR pathway controls the metabolic status of malignant B lymphocytes. Accordingly, the invention relates to a method for determining whether a subject suffering from lymphoma will achieve a response to a treatment with mTOR inhibitor and to a method of treating with an mTOR inhibitor the subject identified as responder.

## Description

### FIELD OF THE INVENTION:

The invention is in the field of cancerology. More particularly, the invention relates to a method for predicting the responsiveness of a patient to a treatment with mTOR inhibitors.

### BACKGROUND OF THE INVENTION:

While tumor cells are extremely diverse with respect to their oncogenic alterations and to their localization, they do share a few common features, including metabolic reprogramming^{1,2}. Initially, it was suggested that most tumor cells produce their energy through aerobic glycolysis due to mitochondrial dysfunction, known as the Warburg effect^{3,4}. However, in recent years, it appears that mitochondria are functional in most tumor cells and actively participate in ATP production through oxidative phosphorylation (OxPhos). The notion that the metabolism of tumor cells is different from that of most normal cells led the field to develop metabolic inhibitors with the intent to kill or to sensitize tumor cells to chemotherapies⁵. Thus far, more than 100 clinical trials are ongoing using metabolic inhibitors in cancer patients⁶. Unfortunately, until now, most of those trials failed to improve outcomes in patients when added to standard cancer therapy⁷. One of the main reasons is that there is not a reliable method to determine the metabolic status of a tumor (glycolytic or OxPhos) in clinic and to determine whether a patient is likely to respond to metabolic inhibitors ^{5,8}. Approximately 90% of aggressive lymphomas originate from B-cells and are classified as diffuse large B-cell lymphomas (DLBCLs), a genetically heterogeneous group of tumors, the most common of which are non-Hodgkin (NH) lymphomas ⁹. This metabolically highly active group of tumors were previously treated with CHOP (cyclophosphamide, hydroxydaunorubicin, Oncovin® and prednisone)¹⁰. Later, a combination of Rituximab (monoclonal anti-CD20 antibody) with CHOP (referred as R-CHOP) demonstrated a benefit for patients, in terms of overall survival (OS) and progression-free survival (PFS)¹¹. However, 40% of R-CHOP-treated DLBCL experienced therapeutic failure or relapse. To date, efforts to capture the molecular heterogeneity of DLBCL have relied on gene expression profiling. In one approach, a classification framework known as cell-of-origin (COO) delineates DLBCL subsets that share components of their transcriptional profiles with normal B-cell subtypes, including Germinal Center B-cell (GCB)-like and Activated B-cell (ABC)-like subtypes ¹². In another approach, comparison of different genetic signatures across DLBCLs highlighted three distinct tumor-intrinsic fingerprints: the BCR/proliferation cluster (BCRDLBCL), displaying up-regulation of genes encoding B-cell receptor (BCR) signaling components and referred to as glycolytic; the OxPhos cluster (OxPhos- DLBCL), which is significantly enriched in genes involved in mitochondrial oxidative phosphorylation (OxPhos); and the host response (HR) tumors, largely characterized by a brisk host inflammatory infiltrate ^{13,14}. Among the glycolytic enzymes, one of them, the glyceraldehyde-3-phosphate dehydrogenase (GAPDH), is of particular interest due to its unique role in the context of lymphoma vascularization ¹⁵. Despite what was initially thought, it appears that GAPDH expression is highly regulated and varies among tumors cells, including human lymphoma cells^{15,16}. However, up to now its role in tumor response to immuno-chemotherapeutic agents is unknown.

### SUMMARY OF THE INVENTION:

The invention relates to a method for determining whether a subject suffering from lymphoma will achieve a response to a treatment with mTOR inhibitors comprising the following steps: i) quantifying the expression level of GAPDH in lymphoma cells obtained from said subject; ii) comparing the expression determined at step i) with a predetermined reference value, and iii) concluding that the subject will achieve a response to a treatment with mTOR inhibitors when the expression level of GAPDH determined at step i) is lower than the predetermined reference value or concluding that the subject will not achieve a response to a treatment with mTOR inhibitors when the expression level of GAPDH determined at step i) is higher than the predetermined reference value. In particular, the present invention is defined by the claims.

### DETAILED DESCRIPTION OF THE INVENTION:

Using primary Eµ-Myc lymphoma cells, inventors observed that Eµ- Myc-GAPDHhigh clones presented less mTOR activity than Eµ-Myc-GAPDHlow clones, as determined by the increase in p70-S6K phosphorylation in the latter. Importantly, inhibition of mTOR with rapamycin in two independent Eµ- Myc-GAPDHlow clones induces a metabolic shift from OxPhos to glycolysis. These results suggest that GAPDH-dependent modulation of the mTOR pathway controls the metabolic status of malignant B lymphocytes.

Accordingly, the invention relates to a method for determining whether a subject suffering from lymphoma will achieve a response to a treatment with mTOR inhibitor comprising the following steps: i) quantifying the expression level of Glyceraldehyde-3-phosphate dehydrogenate (GAPDH) in lymphoma cells obtained from said subject; ii) comparing the expression level determined at step i) with a predetermined reference value, and iii) concluding that the subject will achieve a response to a treatment with mTOR inhibitor when the expression level of GAPDH determined at step i) is lower than the predetermined reference value or concluding that the subject will not achieve a response to a treatment with mTOR inhibitors when the expression level of GAPDH determined at step i) is higher than the predetermined reference value.

As used herein, the term "Glyceraldehyde-3-phosphate dehydrogenate (GAPDH)" refers to an key enzyme of the glycolytic pathway which catalyzes the reaction of glyceraldehyde-3-phosphate (G3P) + NAD⁺ + Pi into 1,3 diphosphoglycerate + NADH + H⁺. The naturally occurring human GAPDH gene has a nucleotide sequence as shown in Genbank Accession number NM_001256799.2 and the naturally occurring human GAPDH protein has an aminoacid sequence as shown in Genbank Accession number NP_001276674.1. The murine nucleotide and amino acid sequences have also been described (Genbank Accession numbers NM_001289726.1 and NP_001276655.1).

As used herein, the term "expression level" refers to the expression level of a gene of interest (e.g. GAPDH). It refers to the expression of the transcripts and/or proteins. The level expression of the gene of interest may in general be determined by either measuring mRNA from the cells and/or measuring expression products, such as proteins. Expression of the transcripts and/or proteins encoded by the nucleic acids described herein may be determined by any of a variety of known methods in the art. The level of GAPDH expression in cells such as lymphoma cells (i.e. B cells) obtained from the patient may be determined using any technique suitable for detecting GAPDH levels in cells. Typically, the level of GAPDH expression is determined by quantitative PCR (qPCR), or immunohistochemistry (IHC). Typically the B cells are obtained from a biopsy, preferably a lymph node biopsy or from a blood sample. Flow cytometry may also be used to obtain B cells. An example of method for measuring the level of GAPDH expression in B cells is: cells are permeabilized and fixed using the BD Cytofix/cytoperm solution (BD Biosciences) and incubated at 4°C for 20 min; the cells are then washed in saponin containing buffer (BD Perm/Wash) and resuspended in the same buffer containing anti-GAPDH antibody (Abcam ab9485; dilution 1/100) and incubated for 30 min at 4°C; the cells are washed twice with the saponin-containing buffer and incubated with a Allophycocyanin (APC)-coupled anti-Rabbit antibody (dilution 1/100) for 30 min at 4°C in the same buffer; after washing twice in the saponin-containing buffer, the cells are resuspended in PBS/2%FCS and analyzed by flow cytometry.

As used herein, the term "subject" refers to any mammals, such as a rodent, a feline, a canine, and a primate. Particularly, the subject is a human. Particularly, the subject is afflicted with lymphoma. As used herein, the term "lymphoma" refers to a group of blood cell tumours that develop from lymphatic cells. The two main categories of lymphomas are Hodgkin lymphomas (HL) and the non-Hodgkin lymphomas (NHL). In the context of the invention, the subject suffers from non-Hodgkin lymphomas. Non-Hodgkin lymphomas, also known as non-Hodgkin refers to a group of blood cancers that include any kind of lymphoma except Hodgkin's lymphomas. Types of NHL vary significantly in their severity, from slow growing to very aggressive types. In a particular embodiment, the subject suffers from a diffuse large B-cell lymphoma (DLBCL) which is the most common non-Hodgkin's lymphoma.

In a particular embodiment, the subject afflicted by lymphoma is treated with mTOR inhibitor. As used herein, the term "mTOR" refers to mammalian target of rapamycin, kinase that in humans is encoded by the mTOR gene. mTOR is a member of the phosphatidylinositol 3-kinase-related kinase family of protein kinases (PI3K). The naturally occurring human mTOR gene has a nucleotide sequence as shown in Genbank Accession number NM_004958.3 and the naturally occurring human mTOR protein has an aminoacid sequence as shown in Genbank Accession number NP_004949.1. The murine nucleotide and amino acid sequences have also been described (Genbank Accession numbers NM_020009.2 and NP_064393.2). mTOR is involved in different pathways, including insulin, growth factors (such as IGF-1 and IGF-2), and amino acids, cellular nutrient, oxygen, and energy levels. In the context of the invention, inventors have shown that the pathway of mTOR is involved in the metabolic status of malignant B lymphocytes. More particularly, in the context of lymphoma, there is an overexpression of PI3K and AKT pathway. The term "mTOR inhibitors" refers to a class of drugs that inhibit mTOR. mTOR inhibitors inhibits cellular metabolism, growth, proliferation, and the formation and signaling through two protein complexes, mTORC1 and mTORC2. More particularly, the mTOR inhibitors inhibit also the PI3K and AKT pathways. mTOR inhibitors are well known in the art. In the context of the invention, mTOR inhibitor is selected from the group consisting of rapamycin and rapalogs (sirolimus; temsirolimus; everolimus; deforolimus); vincristine; dactolisib or BEZ235 (phase I/II of clinical trial; Novartis); alpelisib (BYL719 ,phase III of clinical trial; Novartis); or sapanisertib (phase II of clinical trial; NCI); or taselisib (GDC-0032; phase II of clinicial trial, Roche).

As used herein, the terms "will achieve a response" or "respond" refer to the response to a treatment of the subject suffering from a disorder. Typically such treatment induces, ameliorates or otherwise causes an improvement in the pathological symptoms, disease progression or physiological conditions associated with or resistance to succumbing to a disorder. Accordingly, the survival time of the subject is increased with said treatment. In particular, in the context of the invention, the term "respond" refers to the ability of mTOR inhibitor to an improvement of the pathological symptoms, thus, the subject presents a clinical improvement compared to the subject who does not receive the treatment. The said subject is considered as a "responder" to the treatment. The term "not respond" refers to a subject who does not present any clinical improvement to the treatment with an mTOR inhibitor treatment. This subject is considered as a "non-responder" to the treatment. Accordingly, the subject as considered "non-responder" has a particular monitoring in the therapeutic regimen.

As used herein, the term "predetermined reference value" refers to a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. For example, retrospective measurement in properly banked historical subject samples may be used in establishing the predetermined reference value. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. For example, after determining the expression level of the selected peptide in a group of reference, one can use algorithmic analysis for the statistic treatment of the expression levels determined in samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is high. This algorithmic method is preferably done with a computer. Existing software or systems in the art may be used for the drawing of the ROC curve, such as: MedCalc 9.2.0.1 medical statistical software, SPSS 9.0, ROCPOWER.SAS, DESIGNROC.FOR, MULTIREADER POWER.SAS, CREATE-ROC.SAS, GB STAT VI0.0 (Dynamic Microsystems, Inc. Silver Spring, Md., USA), etc.

In another embodiment, the invention relates to a method for treating a subject suffering from lymphoma in need thereof with an mTOR inhibitor, wherein said method comprises the following steps:
a) identifying whether said subject will achieve a response to treatment with a mTOR inhibitor with the method according to the invention; and
b) treating with a mTOR inhibitor the subject identified as responder.

As used herein, the terms "treating" or "treatment" refer to both prophylactic or preventive treatment as well as curative or disease modifying treatment, including treatment of subject at risk of contracting the disease or suspected to have contracted the disease as well as subject who are ill or have been diagnosed as suffering from a disease or medical condition, and includes suppression of clinical relapse. The treatment may be administered to a subject having a medical disorder or who ultimately may acquire the disorder, in order to prevent, cure, delay the onset of, reduce the severity of, or ameliorate one or more symptoms of a disorder or recurring disorder, or in order to prolong the survival of a subject beyond that expected in the absence of such treatment. By "therapeutic regimen" is meant the pattern of treatment of an illness, e.g., the pattern of dosing used during therapy. A therapeutic regimen may include an induction regimen and a maintenance regimen. The phrase "induction regimen" or "induction period" refers to a therapeutic regimen (or the portion of a therapeutic regimen) that is used for the initial treatment of a disease. The general goal of an induction regimen is to provide a high level of drug to a subject during the initial period of a treatment regimen. An induction regimen may employ (in part or in whole) a "loading regimen", which may include administering a greater dose of the drug than a physician would employ during a maintenance regimen, administering a drug more frequently than a physician would administer the drug during a maintenance regimen, or both. The phrase "maintenance regimen" or "maintenance period" refers to a therapeutic regimen (or the portion of a therapeutic regimen) that is used for the maintenance of a subject during treatment of an illness, e.g., to keep the subject in remission for long periods of time (months or years). A maintenance regimen may employ continuous therapy (e.g., administering a drug at a regular intervals, e.g., weekly, monthly, yearly, etc.) or intermittent therapy (e.g., interrupted treatment, intermittent treatment, treatment at relapse, or treatment upon achievement of a particular predetermined criteria [e.g., pain, disease manifestation, etc.]).

The invention will be further illustrated by the following figures and examples. However, these examples and figures should not be interpreted in any way as limiting the scope of the present invention.

### FIGURES:

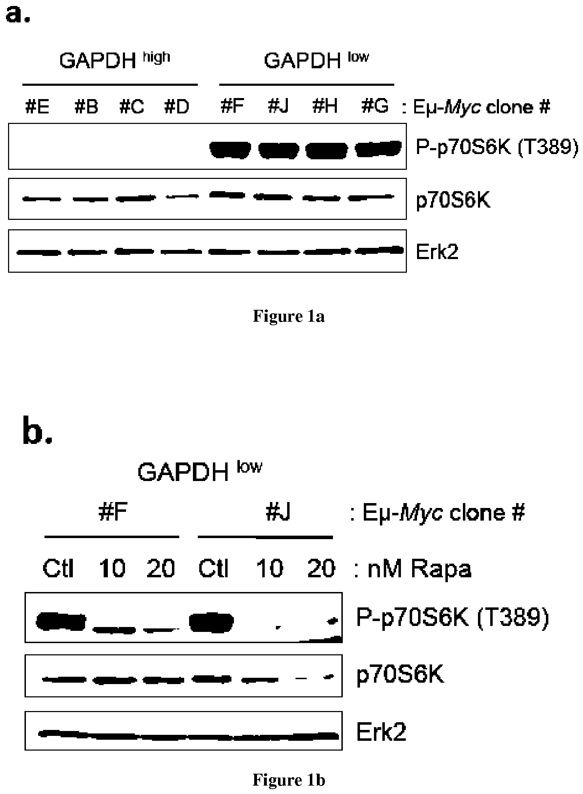

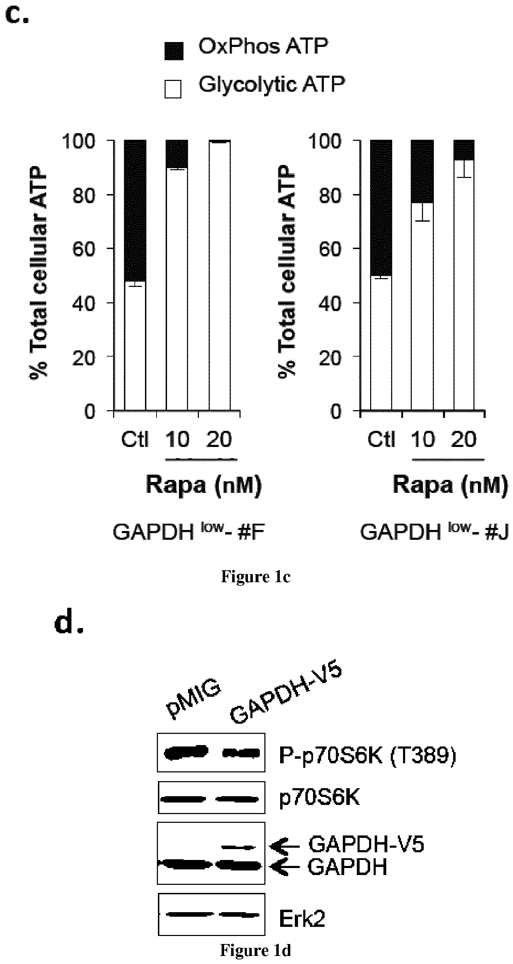

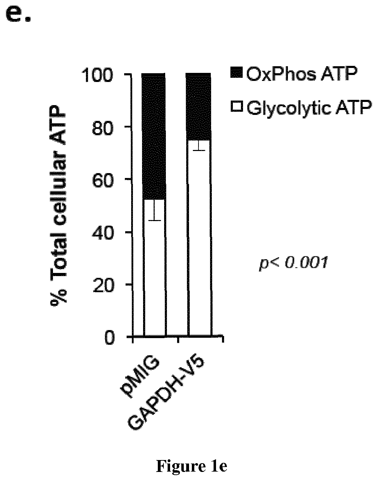

**Figure 1****: GAPDH-dependent control of the mTOR pathway predicts the metabolic status of the tumor.** a. Whole-cell lysates prepared from Eµ-Myc cells harvested from independent Eµ-Myc lymphomas were analyzed by immunoblots with the indicated antibodies. Each lane represents an independent lymphoma. Erk2 is used as a loading control. b. Eµ-Myc-GAPDHlow cells (lymphomas #F and #J) were seeded in the presence or absence (Ctl, DMSO) of 10 nM and 20 nM of Rapamycin for 24 hours. Whole-cell lysates were then analyzed for the indicated proteins. Erk2 was used as a loading control. c. OxPhos and glycolytic ATP production were measured as a percentage of total ATP in Eµ-Myc-GAPDHlow cells harvested from two independent lymphomas (#F and #J) and seeded in the presence or absence (Ctl, DMSO) of 10 nM and 20 nM of Rapamycin for 24 hours. d. Total cell extracts from mouse primary Eµ-Myc-GAPDHlow cells (clone #F) stably transduced with control (pMIG) or GAPDH-V5-encoding pMIG vectors were immunoblotted for the indicated proteins. Erk2 is used as a loading control. e. OxPhos and glycolytic ATP production were measured as a percentage of total ATP in Eµ-Myc-GAPDHlow cells (clone #F) presented in d. Data are shown as means ± SD of 3 independent experiments.

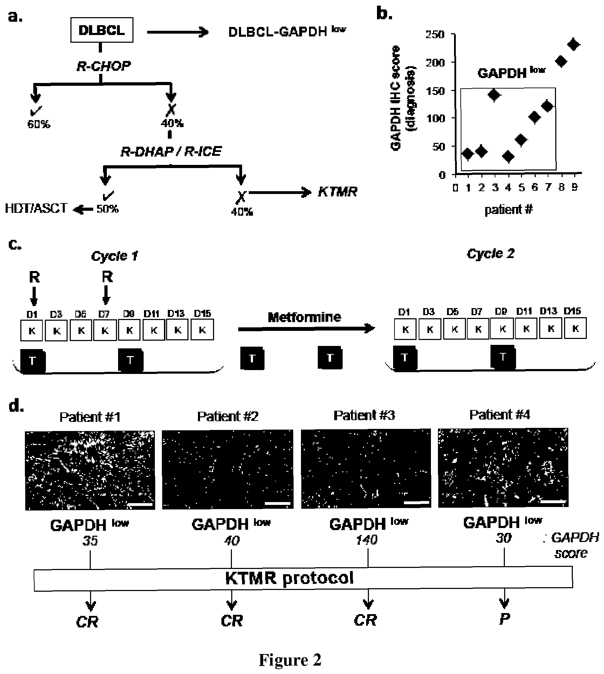

**Figure 2****. Effect of combined mitochondrial inhibitors on the therapeutic response to R-CHOP-refractory DLBCL patients displaying low or high level of GAPDH at diagnosis (clinical trial KTMR).** a. Conventional therapeutic protocol for DLBCL patients. Once diagnosed, the DLBCL patient will receive R-CHOP. About 40% of those patients will not respond to this line of treatment and will benefit from R-DHAP (Rituximab, dexamethasone, cytarabine, and cisplatin) or R-ICE (rituximab, ifosfamidecarboplatin- etoposide) treatment. Finally, another 40% of the patients, will not respond to those chemotherapies and do not benefit from HDT/ASCT (High Dose Therapy with Autologous Stem Cell Transplantation). b. Immunohistochemical score for GAPDH expression in newly diagnosed DLBCL included in the study (n=9). c. Schematic representation of KTMR clinical protocol. R-CHOP refractory DLBCL patients were enrolled in a clinical protocol combining L-asparaginase (Kidrolase, 6000 IU/ml) on days 1, 3, 5, 7, 9, 11 and 13, mTOR inhibitor (Torisel, 75 mg/week) on days 1, 7 and 14, Metformin (1000 mg/day) and anti- CD20 (Rituximab, 375 mg/m2) on days 1 and 7. In between two cycles of treatment patients received Metformin daily (1000 mg/day). d. Illustration of GAPDH expression by IHC in four patients with DLBCL-GAPDHlow biopsies at diagnosis and the therapeutic response to KTMR treatment (CR, complete response; P, progression, respective GAPDH score is indicated). e. Progressive regression of the tumor in patient #1 (CD10+, bcl2+, Mum1-, Ki67+ 80%, MYC+, Ann Arbor Stage IV) upon KTMR treatment.

### EXAMPLE:

**GAPDH-dependent control of the mTOR pathway predicts the metabolic status of the tumors.** In cancer cells, especially upon Myc overexpression 23, glutamine is avidly consumed and used for both energy generation and as a source of carbon and nitrogen for the de novo biosynthesis. Glutamine and other amino acids support mTOR activity, the key sensor of cellular amino acids concentration 24. Importantly, it was recently demonstrated that GAPDH could decrease mTOR activity through its binding to Rheb 25. We therefore investigated whether GAPDH-dependent control of the mTOR pathway was involved in metabolic reprogramming. Using primary Eµ-Myc lymphoma cells, we observed that Eµ- Myc-GAPDHhigh clones presented less mTOR activity than Eµ-Myc-GAPDHlow clones, as determined by the increase in p70-S6K phosphorylation in the latter (Fig. 1a). Importantly, inhibition of mTOR with rapamycin in two independent Eµ- Myc-GAPDHlow clones (Fig. 1b) induces a metabolic shift from OxPhos to glycolysis (Fig. 1c). Consistent with this result, overexpression of V5-tagged GAPDH in Eµ-Myc-GAPDHlow cells reduced mTOR activity, confirmed by the decrease in p70-S6K phosphorylation (Fig 4d), which switched the cells to glycolysis for ATP production (Fig. 1e). These results suggest that GAPDH-dependent modulation of the mTOR pathway controls the metabolic status of malignant B lymphocytes.

**R-CHOP-Refractory DLBCL presenting GAPDHlow expressing tumors at diagnosis, are sensitive to specific metabolic inhibitors.** To ultimately test our hypothesis, we designed an innovative therapeutic strategy to interfere with metabolic reprogramming of R-CHOP refractory DLBCL (Fig. 1a). A local cohort of nine patients who experienced therapeutic failure after a median of 2 prior lines of R-CHOP (range: 2-4, Ann Arbor stage IV, and a high LDH level) was selected (data not shown). Retrospective analysis of GAPDH expression by IHC in de novo DLBCL of those patients confirmed that GAPDHlow tumors (7 out of 9, 78%) were associated with a poor response to RCHOP (Fig. 2b). Treatment consisted in a combination of L-asparaginase (Kidrolase®, Jazz Pharmaceuticals, 6000 UI/m2) on days 1, 3, 5, 7, 9, 11, and 13 of a 4 week-cycle, an mTOR inhibitor (Temsirolimus 75 mg D1, 7, 14), Metformin (1000 mg/day) and Rituximab (375 mg/m2 D1, 7). This therapeutic sequence was called KTMR protocol (Fig. 2c). Three patients completed 4 cycles and one patient 2 cycles with complete response (CR). Five patients completed 1 or less than 1 cycle. The overall response rate was 44% (4/9), and 3 patients achieved a CR. Of the nine patients, three progressed on therapy, and one relapsed 4 months after the end of treatment. The most common reason for discontinuing treatment was progressive disease. Four of the patients presented with toxicities and were removed from the study. Four out of the five patients evaluable for treatment were GAPDHlow and 75% of them (3 out of 4) presented a complete response after two cycles of treatment (Fig. 2d). The overall relapse free survival time of patients benefiting from the KTMR protocol was 4 to 6 months. The effect of the KTMR protocol is illustrated with patient #1 (Fig. 2e), 24 year old man that presented a double-hit Myc/BCL2 germinal center-like diffuse large B-cell lymphoma (DLBCL), with 100% Myc expression and 80% ki67. He had a cervical bulky hypermetabolic mass (diameter 240 x 100 mm) with bone marrow and blood infiltration (but no central nervous system infiltration), an international prognostic index of 2, an Ann Arbor stage of IV, high serum lactate dehydrogenase, and a performance status of 1. The patient had previously received four regimens of imumuno-chemotherapy including RCOPADEM (LMBA protocol; two cycles), and one cycle each of R-CYVE, DAEPOCH- R, and R-ICE. Early tumor progression was systematically observed before initiating each new cycle, demonstrating clearly the extreme chemorefractoriness of his disease. Retrospective analysis of GAPDH expression by IHC showed a low GAPDH score (35) at the diagnosis and, as we predicted according to his GAPDH score, the patient presented resistance to all rituximab-based therapies. After 7 days of treatment following the KTMR protocol, the tumor mass decreased significantly and was dramatically reduced after 15 days of treatment (1 cycle). Finally, 30 days following the beginning of the treatment, he had a reduction of 83% in the tumor mass and was negative at PET-Scan analysis (data not shown). He had a normal life for 4 months and then died upon local and central nervous system relapse of the lymphoma.

Our results also demonstrated that the GAPDH-dependent control of the mTOR pathway dictates the OxPhos or glycolytic metabolic state of the tumor (Fig 1). Consistent with a previous study, it was shown that GAPDH binds Rheb and inhibits mTORC1 signaling. We observed that overexpression of GAPDH inhibits mTOR activity compared to control cells (GAPDHlow) (Fig1). mTOR is known to induce glycolysis through HIF or Myc regulation 35,36. However, it was also reported that mTORC1 inhibition promotes diversion from mitochondrial respiration to aerobic glycolysis 37-39. In our study, it appears that GAPDHlow malignant B cells are primarily OxPhos and that mTOR inhibition leads to impaired mitochondrial function, therefore inducing a metabolic switch to glycolysis in those cells. We observed that GAPDH expression was correlated with the metabolic status of DLBCL. Importantly, we also noted this correlation between gapdh mRNA levels and the metabolic status in follicular lymphomas biopsies, an indolent NH B lymphoma that is treated with R-CHOP (data not shown), suggesting that GAPDH is a marker of the metabolic status of several independent lympho-proliferative malignances.

### REFERENCES:

Throughout this application, various references describe the state of the art to which this invention pertains. The disclosures of these references are hereby incorporated by reference into the present disclosure.

## Claims

1. A method for determining whether a subject suffering from lymphoma will achieve a response to a treatment with mTOR inhibitor comprising the following steps: i) quantifying the expression level of Glyceraldehyde-3-phosphate dehydrogenate (GAPDH) in lymphoma cells obtained from said subject; ii) comparing the expression level determined at step i) with a predetermined reference value, and iii) concluding that the subject will achieve a response to a treatment with mTOR inhibitor when the expression level of GAPDH determined at step i) is lower than the predetermined reference value or concluding that the subject will not achieve a response to a treatment with mTOR inhibitors when the expression level of GAPDH determined at step i) is higher than the predetermined reference value.

2. The method according to claim 1, wherein, the level of GAPDH expression is determined by quantitative PCR (qPCR) or immunohistochemistry (IHC).

3. The method according to claim 1, wherein the mTOR inhibitor is selected from the group consisting of: rapamycin and rapalogs (sirolimus; temsirolimus; everolimus; deforolimus); vincristine; dactolisib or BEZ235; apelisib (BYL719); sapanisertib; or taselisib (GDC-0032).

4. A method for treating a subject suffering from lymphoma in need thereof with an mTOR inhibitor, wherein said method comprises the following steps: a) identifying whether said subject will achieve a response to treatment with a mTOR inhibitor according to the method of claim 1; and b) treating with a mTOR inhibitor the subject identified as responder.
